# EUROPÄISCHE PATENTSCHRIFT

(11) **EP 0 903 107 B1**
(45) Veröffentlichungstag und Bekanntmachung des Hinweises auf die Patenterteilung: **27.07.2005**
(21) Anmeldenummer: 98116384.3
(22) Anmeldetag: 29.08.1998
(51) Int. Cl.: A61B 5/15

(54) **Blutentnahmevorrichtung**
Blood sampling device
Dispositif pour le prélèvement de sang

(30) Priorität: 09.09.1997 DE 19739369
(43) Veröffentlichungstag der Anmeldung: 24.03.1999
(73) Patentinhaber: Sarstedt AG & Co., 51588 Nümbrecht (DE)
(72) Erfinder: Sarstedt, Walter, 51588 Nümbrecht (DE)
(74) Vertreter: Valentin, Ekkehard, Dipl.-Ing.

(56) Entgegenhaltungen:
- US-A- 4 066 067
- US-A- 4 204 606
- US-A- 5 086 783

## Beschreibung

Die Erfindung betrifft eine Blutentnahmevorrichtung, umfassend eine Röhrchenkappe zum Verschließen eines Probennehmerröhrchens, die in einem Dom eine scheibenförmige, durchstechbare, auf einer Auflagefläche aufliegende Membrane aufweist, für die der Dom-Hohlraum zur tiefliegenden Anordnung genutzt und die in einer von der Dom-Oberkante entfernten Position angeordnet ist.

Bei einer durch die DE 29 48 653 C2 bekanntgewordenen Blutentnahmevorrichtung ist die Membrane unmittelbar am oberen Ende des Domes angeordnet; sie wird dort durch eine Bördelung des oberen Domendes oder durch Eindrücken einer zusätzlichen Einlegescheibe in ihrer Lage gesichert und fixiert sowie vorgespannt. Hierbei hat sich allerdings gezeigt, daß dann, wenn ein Blutstropfen auf der am obren Rand des Domes festgespannten Membranscheibe zurückbleiben sollte, was bei der Blutentnahme beim Herausziehen der Probennehmernadel nicht ausgeschlossen werden kann, für die Bedienungsperson die Gefahr eines Kontaktes mit diesem Blutstropfen besteht.

US 4 204 606 A beschreibt eine Röhrchenkappe mit einer festgespannten Membrane und einem nach innen zu einer Wandschürze umgekanteten Domspitzende ; US 5 086 783 beschreibt eine Kappe mit einer scheibenförmigen, durchstechboren Membrane.

Eine die gattungsgemäßen Merkmale aufweisende Weiterbildung der Blutentnahmevorrichtung sieht vor, daß die Membrane in der vorhandenen Bohrung des Domes in einer so tiefliegenden Position eingesetzt ist, daß ein auf der scheibenförmigen Membrane verbleibender Blutstropfen von außen nicht berührt werden kann. Die ortsfeste Lage , d.h. Fixierung und Klemmung der Membrane in dieser tiefliegenden Position wird durch eine seitliche bzw. vom Domumfang her eingeleitete Krafteinwirkung erreicht, indem nämlich der Dom im Bereich der Membranauflagefläche verformt wird. Dennoch läßt sich hierdurch nicht gewährleisten, daß die Blutentnahmevorrichtung im Falle einer Direktadaption dicht bleibt. Bei der Direktadaption werden in maschineller Betriebsweise die Membranen von an- bzw. hintereinandergereihten, mit von Patienten abgenommenen Blut befüllten Röhrchen zur labormäßigen Auswertung des Blutes mit einer Probennehmernadel durchstochen. Wenn diese danach aus der Membranscheibe herausgezogen wird, stellt sich eine - gegenüber einer Nadel bei der Blutentnahme - ungleich höhere Reibung zwischen der Probennehmernadel und der sie im Bereich der Durchstechöffnung umschließenden Membrane ein. Dieser Reibschluß kann es mit sich bringen, daß die Membrane beim Herausziehen der Probennehmernadel ihre Lage verändert und nicht mehr über ihren gesamten Umfang an der Innenwandung des Domes anliegt, womit folglich Blut aus dem Probennehmerröhrchen austreten kann.

Wollte man nun hergehen, die ortsfeste Lage der Membrane durch eine größere Vorspannung beim Einsetzen der Membrane sicherzustellen, ließe sich nicht ausschließen, daß dies zu einer Erlahmung der Membranscheibe führen könnte, wie auch die anfängliche hohe Vorspannung die Eigenschaften der vorzugsweise aus Gummi gefertigten Membrane nachteilig verändern könnte. So wäre insbesondere nicht auszuschließen, daß der Nadel bei der Blutentnahme ein noch höherer Durchdringungswiderstand entgegengesetzt wird, was zu einer höheren traumatischen Belastung des Patienten bei der Blutabnahme, möglicherweise sogar zu einem Durchstechen der Vene führen kann. Im Falle der Direktadaption, d.h. der im Labor maschinellen Entnahme von Blut aus einer Vielzahl neben- und/oder hintereinander angeordneten, mit Blut befüllten Probennehmerröhrchen kommen erschwerend noch andere Eigenschaften hinzu, wie der im Vergleich zu einer Nadel bei der Blutabnahme an einem Patienten sehr viel größere Durchmesser der Nadel, deren Oberflächenbeschaffenheit sowie die Geschwindigkeit beim Ein- und Ausfahren der Direktadaptions-Nadel in die Probennehmerröhrchen. Hier ist die Gefahr noch sehr viel größer, daß die Membrane aus ihrer vorgesehenen Position entfernt, im ungünstigsten Fall sogar ganz aus der Röhrchen- bzw. Verschlußkappe herausgezogen wird; in jedem Fall ist dann ein Auslaufen des Blutes möglich.

Der Erfindung liegt die Aufgabe zugrunde, bei einer gattungsgemäßen Blutentnahmevorrichtung eine Röhrchenkappe ohne die genannten Nachteile zu schaffen.

Diese Aufgabe wird erfindungsgemäß durch die Röhrchenkappe vom Anspruch 1 gelöst. In dieser Vorrichtung ist einerseits die Membrane mit geringstmöglicher, lediglich einen dichtenden Sitz im Dom gewährleistender Vorspannung in der Dombohrung angeordnet und bleibt bei der Blutabnahme in dieser Position und andererseits ist der Dom mit einer am Domspitzenende von außen nach innen umgekanteten, eine sich aufgrund einer höheren Belastung bei der Direktadaption beim Herausziehen einer Probennehmernadel abhebende Membrane in einer eine undichte Schiefstellung verhindernden Lage niederhaltenden Wandschürze ausgebildet. Es wird hierbei von der Erkenntnis ausgegangen, daß eine Festlegung der Membrane mit lediglich einem dichtenden Sitz, wie z.B. durch eine nur noch leichte Verformung des Domes im Bereich der Membranauflagefläche, eine nur noch geringe Krafteinwirkung von außen auf die Domwandung im Bereich der Membranauflagefläche erfordert, so daß keine die leichte Durchdringbarkeit der Membranscheibe beeinträchtigenden Einflüsse bzw. nachteilige Veränderungen der Eigenschaften der Membranscheibe auftreten. Unter dicht wird dabei verstanden, daß beim Einsatz als Vakuumsystem, d.h. der Blutabnahme unmittelbar am Patienten keine Luft von außen in das Röhrchen gelangen und nach der Blutentnahme kein Blut austreten kann.

Sofern es aufgrund der höheren Belastung insbesondere bei der Direktadaption zu einem Abheben bzw. einer Lageveränderung der Membrane kommen sollte, so wird diese dennoch sicher in dem Dom gehalten und kann sich allenfalls nur in eine solche Schieflage verstellen, bei der die Blutentnahmevorrichtung noch dicht ist. Das Spiel bzw. der Abstand zwischen der Schürze, um eine undichte Schieflage auszuschließen, läßt sich auf einfache Weise rechnerisch ermitteln und auf ein Minimum begrenzen. Die Domwand wird von außen nach innen so umgebördelt, daß sie beim Herausziehen der Nadel folglich als sicherer Niederhalter fungiert, vorzugsweise so, daß zwischen dem Ende der Schürze und der Membranscheibe und zwischen Innendurchmesser Dom und Außendurchmesser Schürze ein ausreichend großer Hohlraum bleibt, so daß ein möglicher Blutstropfen in diesen Hohlraum fließen kann.

Die erfindungsgemäßen Maßnahmen ermöglichen gleichzeitig weitere Vorteile bei der Blutabnahme. Denn die den Innendurchmesser des Domes einengende Wandschürze schließt es mit noch größerer Sicherheit aus, daß ein Finger einer Bedienungsperson im Kontakt mit einem auf der Membrane verbleibenden Blutstropfen gelangen kann; dies wäre beispielsweise auch dann gegeben, wenn die Wandschürze statt parallel zu der Domwandung verlaufend rechtwinklig bzw. mit einer geringen Neigungslage nach innen umgekantet würde. Der durch die Wandschürze eingeengte Innendurchmesser unterstützt zudem ein in etwa auf die Mitte zentriertes Einführen einer Probennehmernadel, die mit ihrer angeschärften, spitzen Schneide folglich nicht auf die Auflagefläche der Membrane auftreffen kann, was ansonsten Beschädigungen der Nadelspitze mit sich bringen könnte. Es empfiehlt sich daher, die Einengung des Innendurchmessers abhängig vom Durchmesser der in der Membran-Auflagefläche vorhandenen Durchgangsbohrung zu bemessen, d.h. allenfalls fluchtend mit dieser verlaufen zu lassen, sie demgegenüber jedoch nicht zurückspringend vorzusehen. Schließlich wirkt die Wandschürze auch noch einem möglichen Herausfließen des Blutes aus der Dombohrung entgegen, z.B. wenn das Probennehmerröhrchen mit der aufgesteckten bzw. -geschraubten Röhrchenkappe horizontal auf eine Unterlage abgelegt wird. Das Blut wird dann nämlich in dem zwischen der Schürze und der Domwandung ausgebildeten Ringraum gesammelt. Der Ring- bzw. Hohlraum fängt einen auf der Membrane radial, d.h. nach außen hin wegfließenden Blutstropfen gezielt auf, was durch die Kapillarwirkung schon bei einem geringen Spiel zwischen dem Wandschürzenrand und der Oberfläche der Membrane erreicht wird, und kammert ihn so ein, daß er nicht mehr aus dem Dom heraustreten kann.

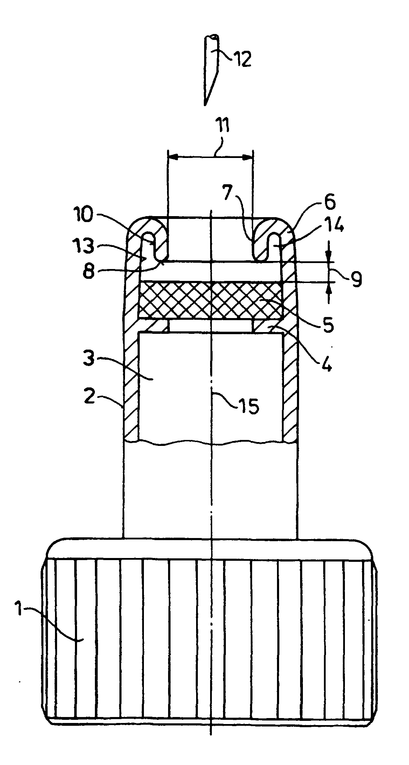

Weitere Einzelheiten und Vorteile der Erfindung ergeben sich aus dem Patentanspruch und der nachfolgenden Beschreibung, in der ein in der einzigen Zeichnungs-Figur in stark vergrößertem Maßstab als Teilansicht schematisch dargestelltes Ausführungsbeispiel des Gegenstandes der Erfindung näher erläutert ist.

Eine auf ein nicht gezeigtes Probennehmerröhrchen von als solche hinlänglich bekannten, nicht dargestellten Blutentnahmevorrichtungen entweder aufschraubbare oder -steckbare Röhrchenkappe 1 weist einen Dom 2 auf, dessen Innenbohrung 3 als Aufnahme für eine darin tiefliegend angeordnete,auf einer Auflagefläche 4 aufliegende Membrane 5 genutzt ist. Von dem oberhalb der Membrane 5 liegenden Domspitzenende 6 ist eine Teillänge zu einer Wandschürze 7 so nach innen umgekantet, daß der freie Wandschürzenrand 8 mit Spiel bzw. einem Abstand 9 von der Oberfläche der Membrane 5 endet.

Das zu der Wandschürze 7 umgekantete Domspitzenwandteil 10 engt die Innenbohrung 3 bis auf einen verbleibenden Kerndurchmesser 11 so ein, daß eine eingeführte Kanüle bzw. Probennehmernadel 12 mit ihrer angeschärften Spitze nicht auf die Auflagefläche 4 auftreffen kann. Gleichzeitig begrenzt es einen unten offenen, zwischen der inneren Domwandung 13 und der dazu in etwa parallel verlaufend umgekanteten Wandschürze 7 ausgebildeten Ringraum 14. Bei einer in der Zeichnungsebene um 90° entweder nach links oder nach rechts, d.h. in Richtung ihrer Längsachse 15 auf eine Unterlage abgelegten Röhrchenkappe 1 kann ein auf der Membranoberfläche möglicherweise verbleibender Blutstropfen radial nach außen bzw. zur Seite fließen und in den Ringraum 14 eintreten, wo er dann daran gehindert wird, aus der Domspitze 2 herauszufließen. Es besteht daher keine Möglichkeit mehr, in Kontakt mit diesem Blut zu kommen.

Der Wandschürzenrand 8 hält aufgrund des Abstandes 9, der sich gegebenenfalls auf ein Minimum verringern läßt, eine sich insbesondere aufgrund einer höheren Belastung bei der Direktadaption beim Herausziehen der Probennehmernadel 12 abhebende Membrane 5 nieder und erlaubt dieser allenfalls eine definierte Schieflage, bei der die Blutentnahmevorrichtung bzw. die Membrane 5 noch dicht ist und die Membrane sicher in der Kappe bzw. der Innenbohrung 3 in ihrer tiefliegenden Position festgehalten wird.

## Patentansprüche

1. Eine Röhrchenkappe (1) zum Verschliessen eines Probennehmerröhrchens für Blutentnahme
mit einer elastischen Membrane (5) und
mit einem Dom (2), der eine Innenbohrung (3), eine Auflagefläche (4) zum Aufliegen der Membrane (5), ein nach innen zu einer Wandschürze (7) umgekantetes sowie ein einen freien Wandschürzenrand (8) bildendes Domspitzende (6) aufweist,
**dadurch gekennzeichnet, dass**
die Membrane (5) scheibenförmig ist, in der Innenbohrung mit einem Abstand (9) zwischen ihrer Oberfläche und dem freien Wandschürzenrand (8) tiefliegt und auf der Auflagefläche (4) derart aufliegt, dass beim Herausziehen einer Probennehmernadel (12) eine undichte Schiefstellung verhindert wird.

## Claims

1. A tube cap (1) for closure of a sampling tube for taking blood comprising an elastic membrane (5) and a mandrel (2) which has an inner hole (3), an abutting surface (4) for resting of the membrane (5), a mandrel tip end (6) inwardly canted towards a wall apron (7) and forming a free wall apron edge (8),
**characterised in that**
the membrane (5) is disk-shaped, lies deep in the inner hole with a distance (9) between its surface and the free wall apron edge (8) and rests on the abutting surface (4) such that when a sampling needle (12) is withdrawn, a leaky oblique position is prevented.

## Revendications

1. Capuchon de pipette tubulaire (1) servant à fermer une pipette tubulaire de prélèvement pour prise de sang
comportant une membrane élastique (5) et
un dôme (2) qui présente un alésage intérieur (3), une surface d'appui (4) pour y poser la membrane (5), une pointe terminale du dôme (6) retournée vers l'intérieur vers un tablier de paroi (7) tout en formant un bord de tablier de paroi libre (8),
**caractérisé en ce que**
la membrane (5) sest en forme de disque, est enfoncée dans l'alésage intérieur (9) avec un écart entre sa surface et le bord de tablier de paroi libre (8) et repose sur la surface d'appui (4) de manière à empêcher, lorsqu'on retire une aiguille de prélèvement (12), une position oblique non hermétique.
